# EUROPEAN PATENT APPLICATION

(11) **EP 2 702 929 A1**
(43) Date of publication of application: **05.03.2014**
(21) Application number: 12776860.4
(22) Date of filing: 12.04.2012
(51) Int. Cl.: A61B 3/024

(54) **CAMPIMETER**

(30) Priority: 25.04.2011 JP 2011096974
(71) Applicant: Kowa Company Ltd., Naka-ku Nagoya-shi Aichi 460-8625 (JP)
(72) Inventor: OOUCHI, Masayoshi, Hamamatsu-shi, Shizuoka 431-2103 (JP); SHIMADA, Satoshi, Hamamatsu-shi, Shizuoka 431-2103 (JP)
(74) Representative: Nobbe, Matthias
(86) International application number: PCT/JP2012/059998
(87) International publication number: WO 2012/147526

(57) **Abstract**

A perimeter (1) having a means (26, 27) for measuring the field of vision of a subject eye by successively causing visual recognition of a visual target (16) projected in different areas, a means (18) for monitoring the fixation status during field of vision measurements, an illumination means (20, 21, 22) for providing illumination to a brightness required for monitoring the fixation status of the subject eye, and a vision correcting means (7) for correcting the vision of a subject eye; wherein the perimeter is provided with: a lens holder part (12) provided to allow a corrective lens on the vision correcting means (7) to be attached, detached, or replaced; a lens brightness database (31) for displaying the brightness of the illumination means when the subject eye (19) is illuminated at essentially the same brightness in accordance with the mode of mounting the corrective lens that is mounted on the lens holder part (12); a means (29) for calculating the state in which the corrective lens is mounted with regard to the vision correcting means (7) ; a means (29) for calculating the brightness of the illumination means corresponding to the mode of mounting of the corrective lens, based on the lens brightness database (31); and a control means (29) for illuminating the subject eye using the illumination means, based on the calculated brightness of the illumination means.

## Description

### TECHNICAL FIELD

The invention relates to a perimeter for measuring a visual field, and especially relates to a perimeter having a visibility corrector, such as a lens holder, to which a correction lens is attachable.

### BACKGROUND ART

A perimeter with a mechanism for locating a visibility corrector, such as a lens for correcting the visibility of a subject eye, has been known, as disclosed in Patent related document 1 mentioned hereinafter.

Perimetry takes a longer time, and a fixation state of a subject eye may be shifted during the perimetry. In such a state, it is not possible to obtain a correct measurement result. For this reason, a fixation state monitor for detecting whether the fixation state of the subject eye is stable has been proposed as disclosed in Patent related documents 2 and 3 mentioned hereinafter. Furthermore, Patent related document 4 discloses such an art that credibility of the measurement value is judged based upon the detected result of the fixation state and such a judgment is set as a standard at a time of reexamination.

### PRIOR ART

### PATENT RELATED DOCUMENT

[Patent related document 1]: Japanese Laid-open Patent Publication No.2003-164425
[Patent related document 2]: Japanese Examined Patent Publication No.S62-009330
[Patent related document 3]: Japanese Examined Patent Publication No.H06-16748
[Patent related document 4]: Japanese Laid-open Patent Publication No.2010-088541

### DISCLOSURE OF INVENTION

### PROBLEMS TO BE SOLVED BY INVENTION

It is desirable to provide the visibility corrector, such as a mechanism in which a lens for correcting the visibility is located, and the fixation state monitor for detecting the fixation state and judging credibility of the measurement result together in the same perimeter. If both are simply provided, a correction lens or the lens holder that supports the correction lens is located between a front eye portion of the subject eye and a fixation state monitor for monitoring the fixation state. Then, due to light emitted from a light source for monitoring the fixation that is located for detecting the fixation state to the front eye portion, a shadow of the correction lens and/or the lens holder is projected to the front eye portion. Such a shadow prevents monitoring of the fixation state and it is not possible to correctly detect the fixation state.

When using the visibility corrector, generally, one or more correction lenses are selected out a group of the correction lenses that are different in the kind of the lens, such as a spherical lens and a cylindrical lens, the degree of the lens and the number of lenses so as to be used by installing in the lens holder. For this reason, the brightness in the position of the eye of the examinee is different according to the installation mode of the lens to be installed in the lens holder. That is, the brightness of the subject eye lighted by the lights emitted from the light source for fixation monitoring on the front eye portion is different, with or the out the correction lens, or depending on the installation mode of the correction lens, and such a fact gives a bad influence on the monitoring action of the fixation state through the fixation state monitor.

Then, an object of the invention is to provide the perimeter for correctly detecting the fixation state without being obstructed by the correction lens or the lens holder supporting the correction lens even if the mechanism for locating the lens for correcting the visibility and the fixation state monitor for detecting the fixation state and judging credibility of a measured value are located at the same perimeter.

Besides, another object of the invention is to provide the perimeter for smoothly monitoring the fixation state through the fixation state monitor by lighting the subject eye with uniform brightness with or without the correction lens or even if the installation mode of the correction lens is different.

### MEANS FOR SOLVING PROBLEMS

A first aspect for solving the above-mentioned problems is a perimeter (1) having:
a visual field measurer (26, 27) that measures a visual field of a subject eye (19) when the subject eye (19) perceives stimuli (16) projected in order at different positions on a projection member (15) :
a fixation state monitor (18) that monitors a fixation state of the subject eye at a time of perimetry;
the fixation state monitor having an illuminator (20, 21, 22) that lights the subject eye with a brightness necessary for monitoring the fixation state of the subject eye; and
a visibility corrector (7) that corrects visibility of the subject eye; the perimeter further comprising:
   a lens holder portion (12) provided on the visibility corrector (7) so as to detachably attach and exchange and combine one or more correction lenses;
   a memory that stores a lens-brightness database (31) showing brightness of the illuminator (20, 21, 22) when lighting the subject eye (19) with almost the same brightness according to an installation mode of the correction lens installed in the lens holder portion (12);
   an installation mode computer (29) that computes the installation mode of the correction lens with respect to the visibility corrector (7) based upon correction information of the subject eye;
   a brightness computer (29) that computes and determines the brightness of the illuminator in association with the installation mode of the correction lens computed through the installation mode computer (29) based upon the lens-brightness database (31); and
   an illumination controller (29) that lights the subject eye through the illuminator based upon the brightness of the illuminator that was computed and determined through the brightness computer.

A second aspect for solving the above-mentioned problem is the perimeter, wherein the visibility corrector (7) is provided being free to be moved with respect to the projection member (15) between a set position (P1) corresponding to a perimetry position (CT) of the subject eye and a stored position (P2) retracted to an outer periphery (15b) of the projection member; further comprising a position detector (13) that detects whether the visibility corrector (7) is at the set position or the stored position.

A third aspect for solving the above-mentioned problem is the perimeter, wherein the illuminator is comprised of two or more light sources (20, 21, 22) that are located respectively different positions, and the illumination controller controls to light the subject eye when the visibility corrector (7) is positioned at the set position (P1) by more light sources (20, 21, 22) rather than a case where the visibility corrector (7) is positioned at the stored position (P2).

A fourth aspect for solving the above-mentioned problem is the perimeter, wherein the illuminator has at least two light sources,
at least one of the light sources is positioned at a position where the subject eye can be lighted via a lens for correcting visibility that is held by the visibility corrector when the visibility corrector is used at the set position;
at least another one light source is positioned at a position where the subject eye can be directly lighted without passing through the lens for correcting visibility that is held by the visibility corrector even if the visibility corrector is used at the set position.

A fifth aspect for solving the above-mentioned problem is the perimeter, wherein the illuminator has at least three light sources (20, 21, 22), and at least one of the light sources is located on a lower hand of a center of the projection member and at least two light sources are positioned at both sides of a lower hand of the projection member.

A sixth aspect for solving the above-mentioned problems is the perimeter, wherein the illumination controller lights the subject eye in a state that the brightness of the light source when the visibility corrector is positioned at the set position is made higher than one when the visibility corrector is positioned at the stored position.

A seventh aspect for solving the above-mentioned problems is the perimeter, wherein the lens-brightness database shows the brightness of the illuminator in association with presence of the lens installed in the lens holder portion, and a kind and a refractive index of the lens.

A eighth aspect for solving the above-mentioned problems is the perimeter, wherein the lens holder portion is provided with a sensor that detects the kind, a degree and the number of the correction lenses installed in the lens holder portion and outputs the detected to the outside, and the installation mode computer computes an installation mode of the correction lens based upon a signal from the sensor.

The ninth aspect for solving the above-mentioned problems is the perimeter, wherein the illuminator has at least two light sources, and the illumination controller selectively drives the light sources when lighting the subject eye.

The tenth aspect for solving the above-mentioned problems is the perimeter, wherein the illuminator has at least two light sources, and the illumination controller drives the light sources with different brightness when lighting the subject eye.

### EFFECTS OF INVENTION

According to the above-mentioned first aspect, the brightness computer (29) computes and determines the brightness of the illuminator corresponding to the installation mode of the correction lens that is computed through the installation mode computer (29) based upon the lens-brightness database (31) and the illumination controller (29) lights the subject eye based upon the brightness of the illuminator that was computed and determined, so that the subject eye (19) is lighted with almost the same brightness with or without the correction lens, irrespective of the installation mode of the correction lens, such as the kind, the number and the refractive index of the lenses and it is possible to properly and smoothly monitor the fixation state through the fixation state monitor (18) thereby.

According to the above-mentioned second aspect, the visibility corrector (7) is provided being free to be moved between the set position (P1) corresponding to the measurement position (CT) of the subject eye and the stored position (P2) retracted to the outer periphery portion (15b) of the projection member, so that it is possible to retract the visibility corrector to the outer periphery portion (15b) that does not interfere with the perimetry when the visibility corrector is not used, and it is convenient thereby.

According to the above-mentioned third aspect, the illumination controller controls to light the subject eye (19) when the visibility corrector (7) is positioned at the set position (P1) by more light sources (20, 21, 22) rather than a case where the visibility corrector (7) is positioned at the stored position (P2), so that lighting is possible so as to cancel the shadow of the visibility corrector projected to the front eye portion due to some light source by another light source and it is possible to light the subject eye (19) in a uniform state with little shadows and to more smoothly monitor the subject eye (19) through the fixation state monitor (18) .

According to the above-mentioned fourth aspect, the subject eye is lighted by at least one illuminator through the lens for correcting visibility that is held by the visibility corrector and is directly lighted by at least one remaining illuminator without passing through the lens for correcting visibility when the visibility corrector is used at the set position, so that it is possible to extremely prevent the shadow of the visibility corrector from being projected onto the subject eye.

According to the above-mentioned fifth aspect, the perimeter has at least three light sources (20, 21, 22), and at least one is located on the lower hand of the center of the projection member and at least two are located on both sides of the lower hand of the projection member, so that the light is emitted on the subject eye 19 from at least three different directions so as to cancel the shadows fallen on the front eye portion of the subject eye 19 by the visibility corrector and the fixation state monitor 18 monitors the fixation state of the subject eye 19 in a good state. Since the light sources (20, 21, 22) are located on the lower hand of the projection member (15), that is, on the lower hand of the perimetry position (corresponding to the center CT) in the projection member (15) of the subject eye (19), the lighting onto the subject eye (19) by the light sources (20, 21, 22) does not interfere with the jaw portion of the upper portion of the subject eye (19) and it is convenient thereby.

According to the above-mentioned sixth aspect, the illumination controller lights the subject eye in a state the brightness of the light source when the visibility corrector is positioned at the set position is made higher than one when the visibility corrector is positioned at the stored position, so that the influence of the visibility corrector that is at the set position with respect to the subject eye is extremely eliminated so as to keep the brightness of the subject eye.

According to the above-mentioned eighth aspect, the sensor is possible to detect the kind, the degree and the number of the correction lenses and the input operation of such data through the keyboard is saved thereby.

According to the above-mentioned ninth and tenth aspects, it is possible to light the subject eye in various modes and to select the proper illumination mode according to the installation mode of the correction lens of the visibility corrector to be used.

The number in parentheses shows the corresponding element in the drawings for the sake of convenience, accordingly, the descriptions are not restricted and bound by the descriptions on the drawings.

### BRIEF DESCRIPTION OF DRAWINGS

[Fig.1] Fig.1 is a front view that shows an example of a perimeter to which the invention is applied.
[Fig.2] Fig.2 is a side view of Fig.1.
[Fig.3] Fig.3 is a front view that shows an example of a lens holder to be installed in the perimeter of Fig.1.
[Fig.4] Fig.4 is a front view that shows a laid down state of a holder body of the lens holder of Fig.3.
[Fig.5] Fig.5 is an enlarged view of a sensor of the lens holder of Fig.3.
[Fig.6] Fig.6 is an enlarged view of the sensor of the lens holder of Fig.4.
[Fig.7] Fig.7 is a front view that shows projection members of the perimeter of Fig.1, such as a visual field dome.
[Fig.8] Fig.8 is a side view of Fig.7.
[Fig.9] Fig.9 is a control block diagram of the perimeter of Fig.1.
[Fig.10] Fig.10 is a side view that shows another example of the visual field dome.
[Fig.11] Fig.11 is a front view that shows yet another instance of the visual field dome.
[Fig.12] Fig.12 is a front view that shows another instance of the lens holder.
[Fig.13] Fig.13 is a view that shows an instance of a lens-brightness table.

### PREFERRED EMBODIMENT

An embodiment of the invention is now explained, referring to appended drawings.

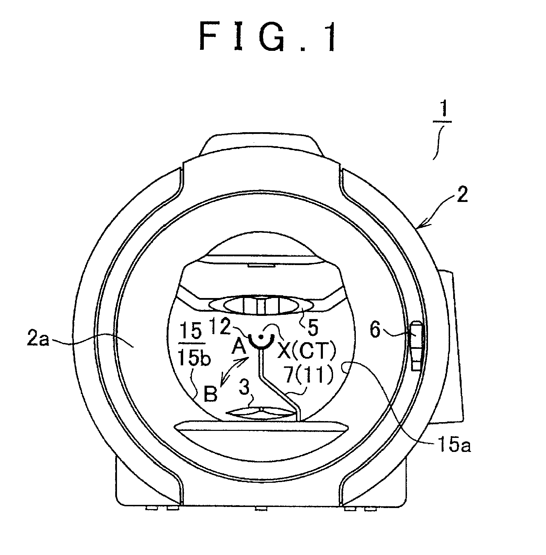

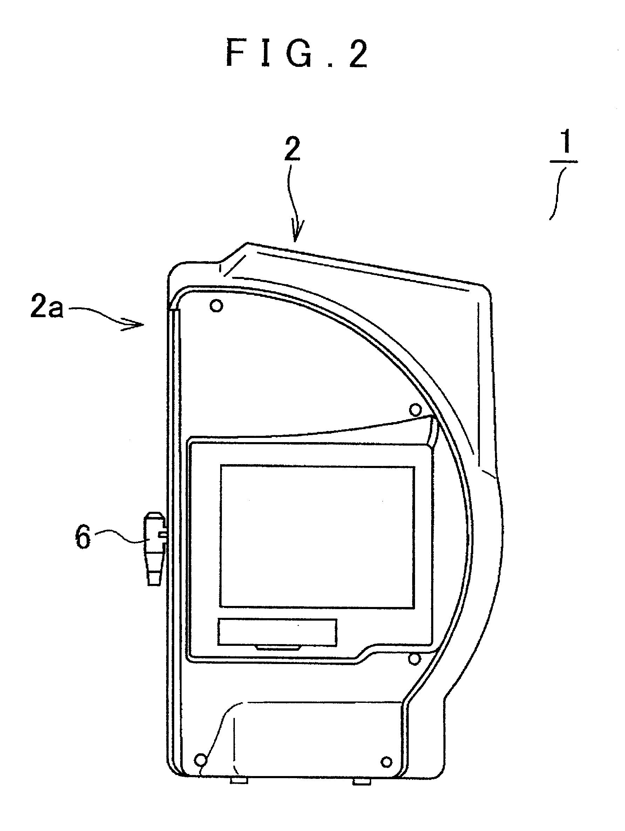

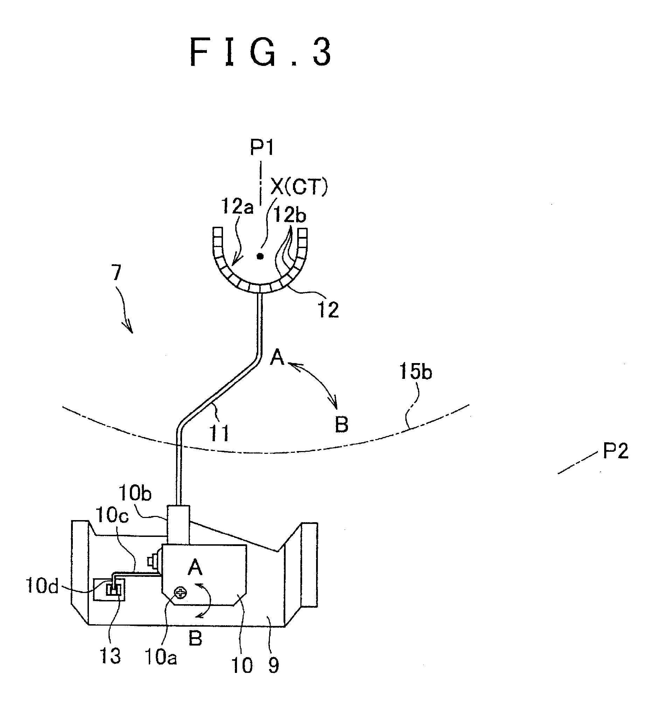

As shown in Figs.1 and 2, a perimeter 1 has a main body 2 the whole of which is in the shape of a box, and a jaw stand 3 and a forehead pad 5 are provided at a front face 2a of the main body 2. A response switch 6 is attachably and detachably located on a right side of Fig.1 of the main body 2, and a lens holder 7 is provided at a back of the paper of Fig.1 of the jaw stand 3. As shown in Fig.3, the lens holder 7 has a bracket 9 that is attached to the main body 2, and a holder body 10 is provided at the bracket 9 so as to go and return in a direction as shown by arrows A and B between a set position P1 and a stored position P2 as shown in Fig.3 with a rotation axis 10a as its center.

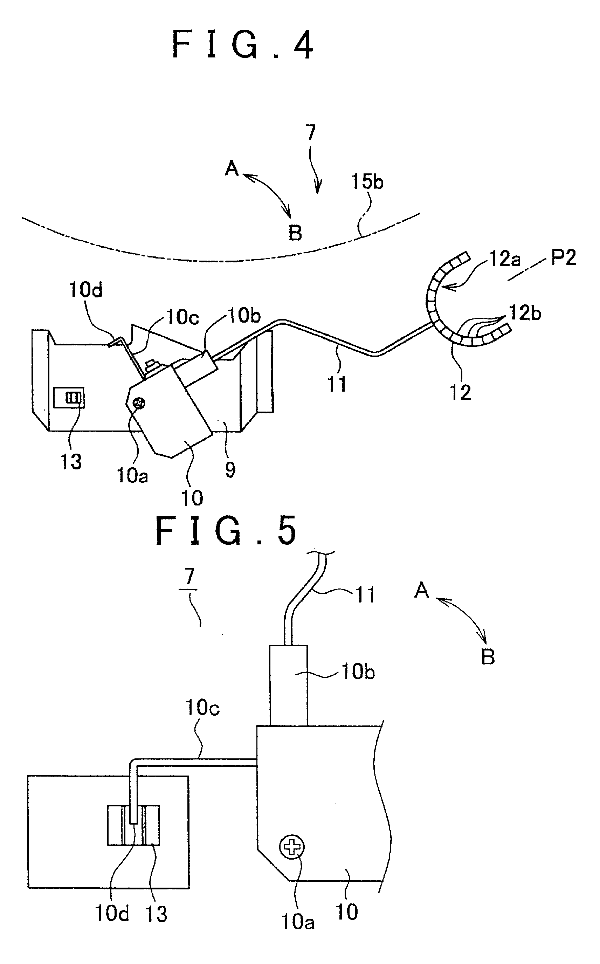

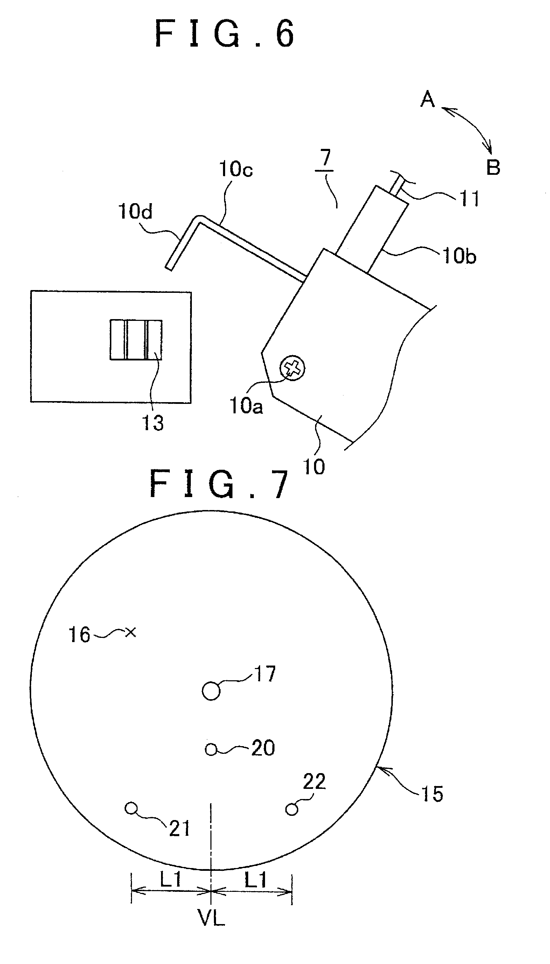

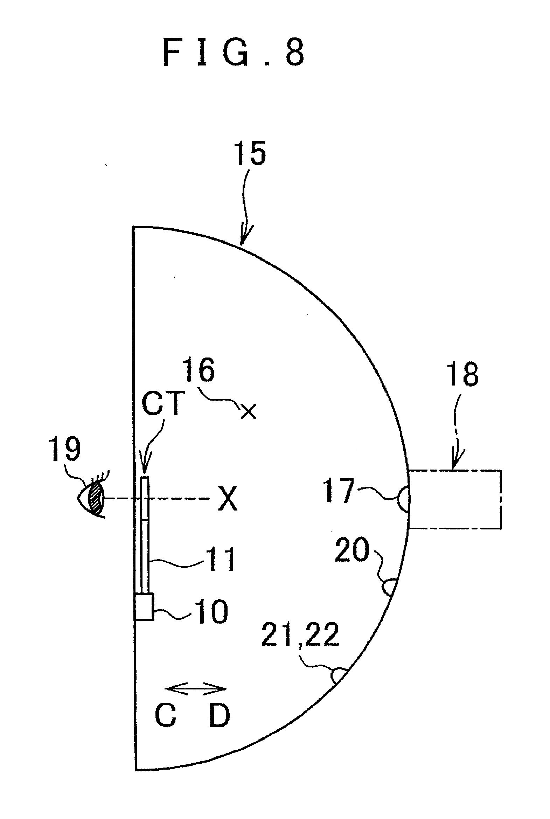

A stem 11 is installed on the holder body 10 through an axial portion 10b that is provided so as to be moved and positioned in a direction perpendicular to the paper of Figs.1 and 3, that is, in a direction parallel to an optical axis X direction of a correction lens installed in a lens holder portion 12 mentioned hereinafter (in a direction as shown by arrows C and D of Fig.8), and the lens holder portion 12 is formed at a top end of the stem 11. And, a concave portion 12a is provided at a top end of the lens holder portion 12. One or more correction lenses for correcting visibility of a subject eye (not shown) are exchangeably and detachably attached to the concave portion 12a, and in a case where the correction lens is one for astigmatism correction, an angle index 12b for adjusting its set angle is provided at the lens holder portion 12. A probe 10c formed in the shape of L character is provided at a left side of Fig.3 of the holder body 10 such that a bent top end 10d is able to be inserted in and pulled out of a lens holder sensor 13 provided at the bracket 9 with a rotation of the lens holder portion 12 in the direction as shown by the arrows A and B, and the lens holder sensor 13 outputs an ON ("1") signal when the top end 10d of the probe 10c is inserted into the lens holder sensor 13 as shown in Fig.5 and outputs an OFF ("0") signal when the top end 10d is pulled out of the lens holder sensor 13 as shown in Fig.6.

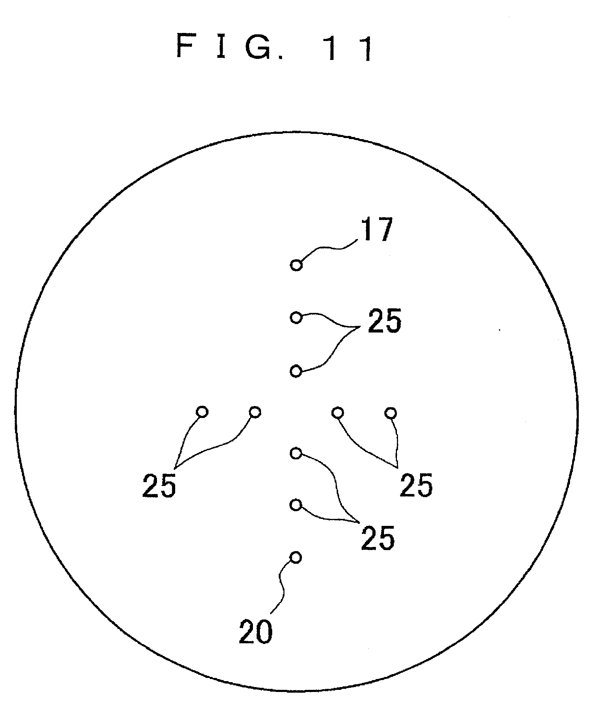

In the main body 2 of a back of the paper of Fig.1 of the lens holder 7, a projection member, such as a visual field dome 15 in a semi-spherical shape through which stimuli are presented, is provided, and as shown in Figs.7 and 8, a stimulus 16 for perimetry can be presented at an optional position in the visual field dome 15 through a well-known stimulus presenter mentioned hereinafter. A fixation lamp 17 that is a target when a subject eye fixates is provided at an innermost portion of the dome 15 with respect to the front face 2a of the main body in the visual field dome 15, and a well-known fixation state monitoring device 18 for watching a fixation state of a subject eye 19 is provided at the back of Fig.7 of the fixation lamp 17, that is, on a right hand of Fig. 8 such that the fixation state of the subject eye 19 can be monitored at the time of perimetry. In some cases, the fixation lamp has an auxiliary fixation lamp which is comprised of two or more LEDs 25 located in the shape of a cross in the visual field dome 15, as shown in Fig.11 for the examinees who are difficult to fixate the center or in order to chiefly perform the inspection of the peripheral portion of the visual field, as well as the fixation lamp 17 and the LED illumination 20.

Three LED illuminations 20, 21 and 22 are located on a lower hand of Fig.7 of the fixation lamp 17 of the visual field dome 15, that is, on the visual field dome 15 on an obliquely lower hand of Fig.8 of the subject eye 19 to be measured. The LED illumination 20 is located on a lower hand in the vertical direction of the fixation lamp 17 that is a center position (that is, the lower hand of the center portion of the visual filed dome 15), and the remaining two LED illuminations 21 and 22 are located at symmetrical positions in a right/left direction with respect to a vertical face VL passing the fixation lamp 17 and the LED illumination 20 on the lower hand of the fixation lamp 17 (that is, both sides of the lower hand of the visual field dome 15) such that each of both symmetrical positions is apart from the vertical face VL by a predetermined distance L1.

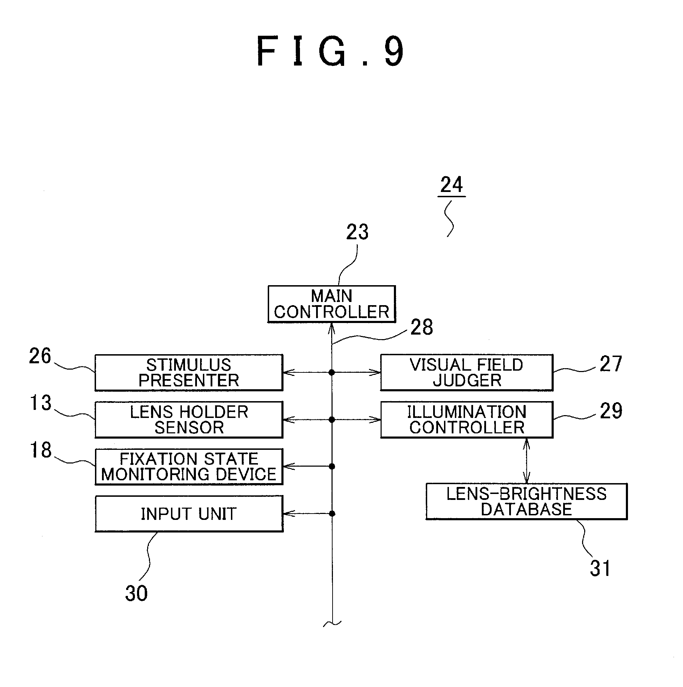

As shown in Fig. 9, a controller 24 of the perimeter is provided at the main body 2 of the perimeter 1, and the controller 24 has a main controller 23. A stimulus presenter 26, a visual field judger 27, the above-mentioned lens holder sensor 13, an illumination controller 29, the fixation state monitoring device 18 and an input unit 30, such as a keyboard, are connected with the main controller 23 via a bus line 28. And, a lens-brightness database 31 stored in a memory means is connected with the illuminationcontroller29. A control block diagram as shown in Fig.9 shows only portions pertinent to the invention and the other structural portions of the perimeter 1 having no connection with the invention are not shown.

The perimeter 1 has the above-mentioned structure. In a case where the visual field of the subject eye 19 is measured and it is not necessary to adjust the visibility of the subject eye 19, an examiner rotates the lens holder 7 in the arrow B direction with the rotation axis 10a as its center as shown in Fig.4 so as to position the stem 11 and the lens holder portion 12 at the stored position P2. Then, the stem 11 and the lens holder portion 12 are rotated and moved in the arrow B direction from the front face portion as shown in Fig.1 of the perimeter 1, and such a last state that the lens holder portion 12 is located at the set position P1 of the center portion of an opening 15a of the visual field dome 15 (see Fig.3), that is, a position corresponding to a measurement position (almost central position of the visual field dome 15) of the subject eye where the subject eye 19 is located at the time of perimetry is changed into such a state that the stem 11 and the lens holder portion 12 are retracted and stored at the stored position P2 of the lower hand of an outer periphery 15b of the front face of Fig.1 of the opening 15a (see Fig.4), and in such a state, the stem 11 and the lens holder portion 12 are not present (are not watched) at the opening 15a as shown in Fig.1 having almost circular shape when viewing from the direction perpendicular to the paper of Fig.1.

In the afore-mentioned state, an examinee is invited to put his (her) jaw on the jaw stand 3 and contact his (her) forehead with the forehead pad 5 so as to be pressed against such a pad such that the subject eye 19 of a front eye portion of the examinee is located at a predetermined perimetry position, that is, at an almost central position CT of the visual field dome 15 as shown in Fig.8. If the perimeter 1 is instructed to start perimetry of the subject eye 19 through an operation portion, such as a keyboard (not shown) in the afore-mentioned state, the main controller 23 of the controller 24 presents stimuli 16 in order at proper positions in the visual field dome 15 through the stimulus presenter 26 with a well-known method. When the examinee perceives the presented stimulus through the subject eye 19, the examinee operates the response switch 6 and when not, no operation of the response switch 6 is done. The visual field judger 27 measures the visual field of the subject eye with a well-known method, relating the operation state of the response switch 6 and the stimulus position at such a time with each other.

On this occasion, in order to properly conduct the perimetry, the main controller 23 gets the fixation state monitoring device 18 to watch whether the subject eye 19 always fixates the fixation lamp 17 during the perimetry, that is, whether the fixation state is maintained. Such a method of watching the fixation state is already well known, so its detailed explanation is not mentioned. At a time of monitoring the fixation state, the main controller 23 drives the three LED illuminations 20, 21 and 22 through the illumination controller 29 in order to properly monitor the fixation state by the fixation state monitoring device 18 and controls such a driving state such that the subject eye 19 on which the perimetry is conducted can receive lights having brightness proper for the fixation state monitoring for watching whether the subject eye 19 is in the fixation state necessary for the perimetry.

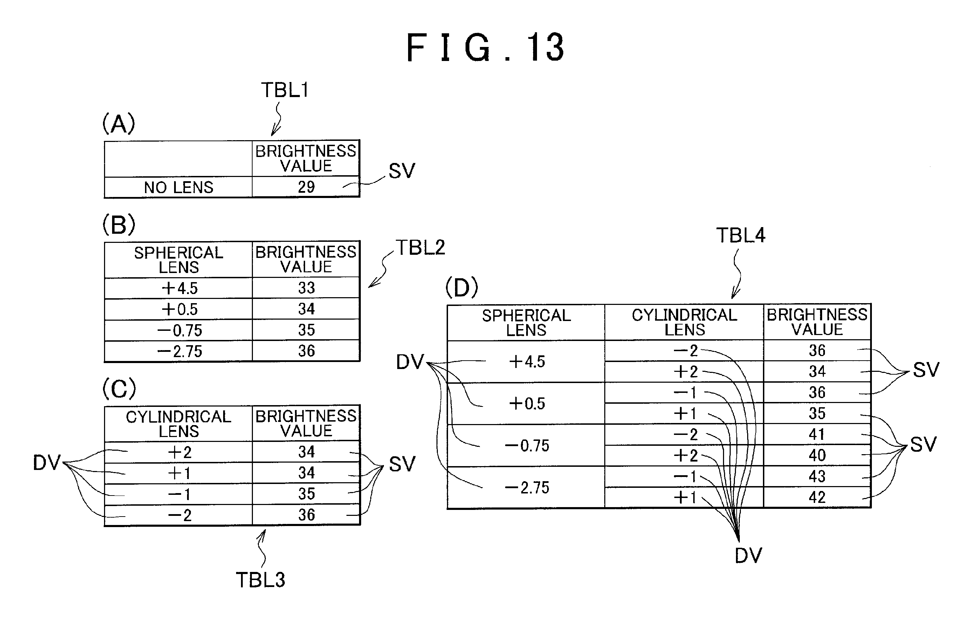

That is to say, the illumination controller 29 monitors an output state of the lens holder sensor 13, and if the output of the lens holder sensor 13 is an OFF signal, that is, in such a case of "no lens" where the lens holder portion 12 of the lens holder 7 is stored in the stored position P2 as shown in Fig.4, the illumination controller 29 searches a lens-brightness table TBL stored in the lens-brightness database 31 and reads a brightness value SV shown in the table TBL1, referring to the lens-brightness table TBL1 in the "no lens" state (no lens table TBL1) as shown in Fig.13(A) so as to determine "29" that is the read brightness value SV as the driving brightness of the LED illuminations 20, 21 and 22. The data shown in such a lens-brightness table TBL are the data set in advance based upon experiments so as to light the subject eye 19 and its periphery with approximately the same brightness by driving the respective LED illuminations 20, 21, 22 with the brightness value SV shown in the lens-brightness table TBL through the illumination controller 29 regardless of installation modes of the correction lens, such as with or without the lens holder 7, and kinds or a combination of the correction lenses that are installed in the lens holder 7. The lens-brightness database 31 will be mentioned later in detail.

After reading the brightness value SV for driving the LED illuminations 20, 21 and 22 in the "no lens" state out of the lens-brightness database 31 and determining such a value through the illumination controller 29, the illumination controller 29 drives the LED illuminations 20, 21 and 22 in the center of Fig.7 with the brightness value "29" designated in the no lens table TBL1. A method of driving the respective LED illuminations 20, 21 and 22 is optional, and only LED illumination 20 may be driven with a predetermined brightness value designated in the lens-brightness database so as to light the subject eye 19 or the LED illuminations 21 and 22 on both sides may be driven with the LED illumination 20 being in non-driving state. That is, any driving method of the LED illumination is available as long as the subject eye 19 and its periphery are lighted based upon the lens-brightness database with the brightness that does not disturb the fixation monitoring through the fixation state monitoring device 18 according to the installation mode of the LED illuminations and the number of the LED illuminations located.

When thus driving all LED illuminations 20, 21 and 22 or selectively driving the LED illuminations 20, 21 and 22 with the brightness value SV shown in the no lens table TBL of the lens-brightness database 31, the subject eye 19 and its periphery are lighted by the LED illuminations driven with the brightness value "29". In such a state, the lens holder portion 12 is at the stored position P2 stored on a back side of the front face 2a on the lower hand of the outer periphery 15b of the visual field dome 15 of Fig.1 with the stem 11, as mentioned before, and the lens holder 7 does not disturb the monitoring through the fixation state monitoring device 18, and it is possible to sufficiently monitor the fixation state of the subject eye 19 even with the lighting by the LED illuminations the light emission of which is controlled with the low brightness such as the brightness value "29". In a case of this embodiment, the illumination controller 29 can adjust the brightness of the LED illuminations 20, 21 and 22 with 256 phases (0 to 255 levels), and in a case of "no lens" state that the lens holder portion 12 is stored at the stored position P2, the illumination controller 29 drives the LED illumination 20 with brightness value "29", that is, with the brightness "29" level so as to light the subject eye 19.

In a case where it is necessary to adjust the visibility of the subject eye 19, the examiner rotates the lens holder 7 in the arrow A direction with the rotation axis 10a as its center as shown in Fig.3 so as to position the stem 11 and the lens holder portion 12 from the last stored position P2 to the set position P1. Then, the lens holder 7 is positioned so as to locate the concave portion 12a of the lens holder portion 12 at a center of the dome viewed from the front face of Fig.1 of the front face of the visual filed dome 15 (corresponds to an optical axis X of the correction lens), as shown in Figs.1 and 8. In such a state, one or more lens for correcting visibility (not shown) that are suitable for the visibility of the subject eye 19 are installed on and held by the concave portion 12a of the lens holder portion 12, and the axial portion 10B is moved and adjusted in the direction as shown by the arrows C and D of Fig. 8, fitting the visibility of the subject eye 19, and in a case of the correction lens for the astigmatism, such as a cylindrical lens, the lens for correcting the visibility is rotated in the concave portion 12a, referring to the angle index 12b in order to adjust its setting angle such that the subject eye 19 is able to clearly perceive the stimulus 16.

When the lens holder 7 is positioned at the set position P1, the top end 10d of the probe 10c is inserted in the inside of the lens holder sensor 13 as shown in Fig.5 and the ON ("1") signal is outputted from the lens holder sensor 13. When perimeter 1 is instructed through the input device 30, such as a keyboard to start conducting perimetry on the subject eye 19 in the afore-mentioned state, the main controller 23 of the controller 24 presents the stimuli 16 at proper positions in the visual field dome 15 in order through the stimulus presenter 26 with a well-known method.

On this occasion, the main controller 23 gets the fixation state monitoring device 18 to monitor whether the subject eye 19 always fixates the fixation lamp 17 during the perimetry, that is, whether the fixation state is maintained in order to properly conduct the perimetry. But, at this time, the lens holder 7 installing the lens for correcting visibility is located just before the subject eye 19, this time is different from the case where the lens holder 7 is at the stored position P2. That is, when only driving the LED illuminations 20, 21 and 22 with the low brightness, the brightness value SV "29", the shadows of the stem 11 and the lens holder portion 12 of the lens holder 7 and the lens for correcting the visibility installed in the lens holder portion 12 are thrown on the front eye portion of the subject eye 19, and the subject eye 19 and its periphery darken. For this reason, the fixation state monitoring device 18 may not sufficiently monitor the fixation state of the subject eye 19.

At a time when the output of the lens holder sensor 13 turns ON ("1") signal, that is, the lens holder 7 is positioned at the set position P1, the illumination controller 29 searches the lens-brightness table TBL stored in the lens-brightness database 31 so as to compute and determine the brightness of the LED illuminations 20, 21 and 22 suitable for the correction lens installed in the lens holder potion 12 when lighting the subject eye 19.

That is, as shown in Fig.13, the lens-brightness table TBL stored in the lens-brightness database 31 stores the no-lens table TBL1, a spherical lens table TBL2, a cylindrical lens table TBL3 and a combination lens table TBL4 showing the brightness value SV when driving the LED illuminations 20, 21 and 22 every each refractive index of lens (diopter) DV, the no-lens table TBL1 showing the brightness value SV (that is, the brightness) of the LED illuminations 20, 21, 22 in the installation mode "no lens" wherein no correction lens is installed in the lens holder 7 (or in a case where the lens holder 7 is at the stored position P2), the spherical lens table TBL2 showing the brightness value SV of the LED illuminations 20, 21 and 22 when the installation mode of the correction lens installed in the lens holder 7 is the spherical lens, the cylindrical lens table TBL3 showing the brightness value SV of the LED illuminations 20, 21 and 22 when the installation mode of the correction lens installed in the lens holder 7 is the cylindrical lens, the combination lens table TBL4 showing the brightness value SV of the LED illuminations 20, 21 and 22 when the installation mode of the correction lens installed in the lens holder 7 is the combination of the spherical lens and the cylindrical lens. On this occasion, the kinds and the combination of the refractive index DV and the brightness value SV in the lens-brightness table TBL, and the kinds of the correction lenses comprising the lens-brightness table TBL and their combination are illustrative only, and a proper lens-brightness table TBL is prepared in advance according to the real measurement state and is stored in the lens-brightness database 31 (such as, at a time when manufacturing the perimeter or at a time of set-up when the perimeter is firstly used). In this case, + shown before the refractive index of each lens means the refractive index of a lens for hypermetropia and - means the refractive index of the lens for myopia. That is, the lens-brightness database 31 shows the brightness of the LED illumination (that is, the brightness value SV) corresponding to the installation mode of the correction lens to be installed on the lens holder portion 12, with or without the lens, or depending on the kind of the lens, such as the spherical lens and the cylindrical lens, and the refractive index.

As already mentioned before, the driving mode of each LED illumination 20, 21 or 22 is optional, and it is possible to light only LED illumination 20 with a predetermined brightness value designated by the lens-brightness database or to drive the LED illuminations 21 and 22 at both sides with the LED illumination 20 being in non-driving state. That is, any mode of driving the LED illumination is available as long as the subject eye 19 and its periphery are lighted based upon the lens-brightness database with the brightness that does not disturb the fixation monitoring through the fixation state monitoring device 18 according to the installation mode of the LED illuminations and the number of the LED illuminations located (number of the LED illumination may be one). When driving the respective LED illuminations 20, 21 and 22 with the respective brightness values SV, the brightness value SV shown in each lens-brightness table TBL is shown on each LED illumination 20, 21 or 22.

The illumination controller 29 computes and judges the brightness values SV of the respective LED illuminations 20, 21 and 22 in such a way that when searching the lens-brightness table TBL stored in the lens-brightness database 31, the installation mode, such as the kind and the degree of the lens to be installed on the lens holder 7, and number of such lenses, are computed from correction information of the subject eye 19 shown in patient information, such as the degree of the spherical surface and the degree of the column, that was inputted in advance by the examiner through the input unit 30 (the input means of the correction information), and its computed result is applied to each lens-brightness table TBL. The input means of the correction information of the subject eye 19 are a card reader for reading data storing the correction information of the subject eye 19, and a communication controller for downloading the correction information from outside database storing the correction information of the subject eye 19 into the perimeter 1 through a communication line, in addition to the input unit 30. It is possible for the illumination controller 29 to easily compute the installation mode of the correction lens (including such a judgment that the correction lens is not necessary to be used), such as the kind and the degree of the lens to be installed on the lens holder 7, and number of such lenses, from the correction information of the subject eye 19, such as Spherical diopter power and Cylindrical diopter power with a well-known method. It may be configured that a sensor through which the kind, the degree and the number of the correction lens installed on the lens holder portion 12 is detected and a corresponding signal is outputted to the outside is installed, and the illumination controller 29 directly computes the installation mode of the correction lens, such as the kind, the refractive index and the number of the lens installed on the lens holder 7 based upon the signal without depending on the input of the correction information of the subject eye 19. When the lens holder 7 is at the stored position P2, the illumination controller 29 computes and judges the installation mode of the lens to be "no lens".

In a case where the illumination controller 29 judged that the installation mode of the correction lens installed on the lens holder 7 is one spherical lens the refractive index DV of which is -0.75 and the cylindrical lens the refractive index DV of which is +2, the corresponding combination of the lenses in the combination lens table TBL4 of Fig.13(D) is referred so as to compute and determine the brightness value SV of the LED illumination, that is, the brightness as "40". In a case where the illumination controller 29 judged that the installation mode of the correction lens installed on the lens holder 7 is one spherical lens the refractive index DV of which is +4.5, the corresponding lens in the spherical lens table TBL2 of Fig.13(B) is referred so as to compute and determine the brightness value SV of the LED illumination as "33". In a case where the illumination controller 29 judged that the installation mode of the correction lens installed on the lens holder 7 is one cylindrical lens the refractive index DV of which is -2, the corresponding lens in the cylindrical lens table TBL3 of Fig.13(C) is referred so as to compute and determine the brightness value SV of the LED illumination as "36".

When the brightness value SV of the LED illumination according to the kind, the degree and the number of the correction lens installed on the lens holder 7 was thus determined by searching each lens-brightness table TBL of the lens-brightness database 31, the illumination controller 29 drives the LED illuminations 20, 21 and 22 of Fig.7 with the determined brightness value SV "41", for instance. A level of such a brightness value SV "41" is higher than the brightness value SV "29" in the case of no lens on the lens holder 7, and is high brightness. Then, the respective LED illuminations 20, 21 and 22 are driven with the brightness higher than the state having no lens on the lens holder 7. As mentioned before, the driving mode of each LED illumination 20, 21 or 22 can be optionally set. For example, it is possible to light the three LED illuminations 20, 21 and 22 with different brightness, to drive the LED illumination 20 of the center with the brightness higher than the brightness value SV "41" and to drive the LED illuminations 21 and 22 on both sides with brightness lower than the brightness value SV "41", or to drive the LED illuminations in the opposite mode, and furthermore, to selectively drive the LED illuminations 20, 21 and 22. The driving modes of the respective LED illuminations on this occasion and the brightness values SV at the time of driving are stored in the lens-brightness database 31 as driving data of each LED illumination, and the illumination controller 29 drives the respective LED illuminations 20, 21 and 22 selectively and/or with the designated brightness value SV based upon the lens-brightness database 31.

The illumination controller 29 thus lights the subject eye 19 which looks into the visual field dome 15 from three hands, the lower hand of the center and the lower hands on both side of the center. Then, the lights are emitted onto the subject eye 19 from the different three directions, and such lights operates so as to negate the shadows thrown by the stem 11 or the lens holder portion 12 of the lens holder 7, and the lens for correcting the visibility on the front eye portion of the subject eye 19, so that the subject eye 19 and its periphery are lighted with the brightness similar to the case where the correction lens is not installed on the lens holder 7 and the fixation state monitoring device 18 monitors the fixation state of the subject eye 19 in a good state.

Especially, in a case of the three LED illuminations, since all LED illuminations 20, 21 and 22 emit lights onto the subject eye 19 from the obliquely lower direction, the shadows of the lens for correcting the visibility and the lens holder portion 12 that are positioned at almost central position CT are extremely prevented from being projected onto the front eye portion that is on the optical axis Z of the lens for correcting the visibility and it is convenient. Since the LED illuminations 20, 21 and 22 are located on the lower side of the visual field dome 15, that is, on the lower hand of a perimetry position (corresponding to the center CT) in the visual field dome 15 of the subject eye 19, the illumination onto the subject eye 19 through the LED illuminations 20, 21 and 22 is not hindered by the forehead portion of the upper portion of the subject eye 19 and it is convenient. But, this does not prevent the LED illumination for lighting the subject eye 19 from being located on the upper side of the perimetry position of the subject eye 19 in the visual field dome 15.

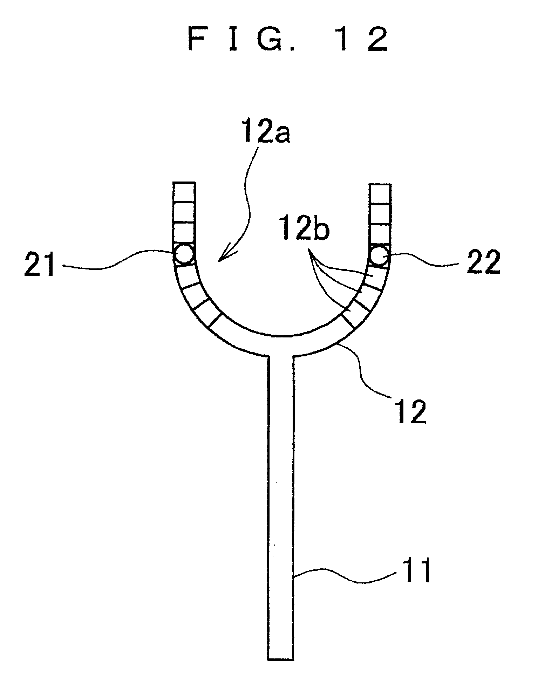

As already mentioned before, the optional number of the LED illuminations, that is, one or more may be located. In a case where two or more LED illuminations are provided, the location mode is optional. For example, as shown in Fig.12, the LED illuminations 21 and 22 may be located on both sides of the concave portion 12a of the lens holder 7 so as to light the subject eye 19 from a neighboring distance.

Emitting light of illumination means onto the subject eye 19 through the LED illuminations 20, 21 and 22 may be infrared light as well as visible light, and visible light and infrared light may be used together. The brightness of the respective LED illuminations 20, 21 and 22 is adjustable, and the brightness of the illumination onto the subject eye 19 can be also adjusted in such a manner that the illumination controller 29 automatically adjusts the brightness or an examiner manually adjusts the brightness according to a state of capturing the subject eye 19 through the fixation state monitoring device 18, such as a contrast of images of the subject eye 19 and a reflected state of Purkinje image.

In a case of the illumination means that is comprised of two or more light sources located at respectively different positions, such as the LED illuminations 20, 21 and 22, the illumination controller 29 controls to light the subject eye 19 when such a visibility corrector, such as the lens holder 7, is positioned at the set position P1 through more light sources than a case where a visibility corrector is positioned at the stored position P2, so that lighting is possible so as to cancel the shadows of the visibility corrector projected onto the front eye portion due to some light source by another light sources. Then, it is possible to light the subject eye 19 in a uniform state with little shadows and to more smoothly monitor the subject eye 19 through the fixation state monitoring device 18.

In a case where the lens holder 7 is used at the set position P1, the use of the lens holder 7, that is, the use of the lens for correcting the visibility at the time of perimetry, is stored in the measurement data by the visual field judger 27 or the fixation state monitoring device 18 based upon the output of the lens holder sensor 13 as attribution data.

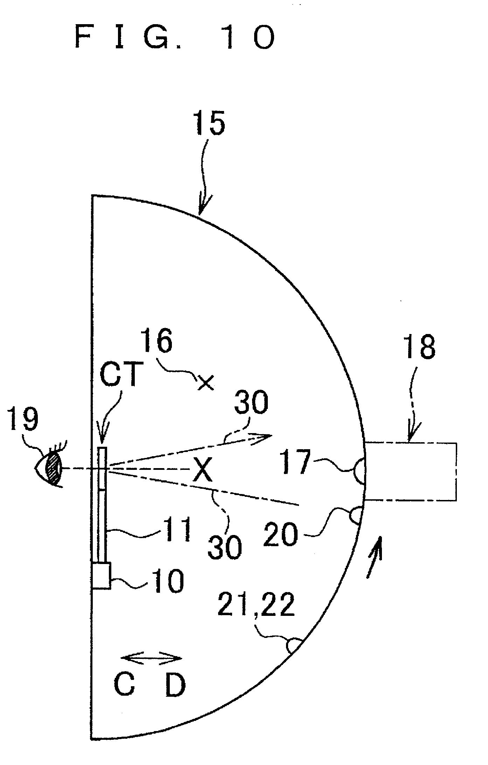

Besides, the LED illumination 20 located on a lower hand of the fixation lamp 17 may be located on a side near the fixation lamp 17 as shown in Fig.10, that is, near the central portion of the visual field dome 15 such that light 30 emitted from the LED illumination 20 can light the subject eye 19 via the lens for correcting visibility that is held by the lens holder portion 12 when the lens holder 7 is positioned at the set position P1. On this occasion, the light 30 from the LED illumination 20 is emitted onto the subject eye 19 via the lens for correcting visibility from the slightly obliquely lower direction rather than a horizontal direction as shown in Fig.10. Even if a part of the light 30 from the LED illumination 20 reflects to the lens for correcting visibility, the reflected light 30 proceeds in a slightly obliquely upper direction of Fig.10 and is prevented from being emitted directly to the fixation state monitoring device 18. Therefore, monitoring of the fixation state through the fixation state monitoring device 18 is not obstructed by the reflected light from the LED illumination 20.

At such a time, the LED illuminations 21 and 22 on the lower hand of Fig.10 are able to light the subject eye 19 directly from a direction lower than the lens for correcting visibility held by the lens holder portion 12 without passing through the lens for correcting visibility, so that even if the shadows of the lens holder 7 and the lens for correcting visibility are projected onto the subject eye 19 through the LED illumination 20, the lighting is possible, canceling such shadows by the illumination from the lower direction through the LED illuminations 21 and 22 and it is convenient.

### EXPLANATION OF REFERENCE NUMBERS

1 perimeter
7 visibility corrector (lens holder)
13 position detector (lens holder sensor)
15 projection member (visual field dome)
15b outer periphery (outer periphery)
16 stimulus
18 fixation state monitor (fixation state monitoring device)
19 subject eye
20, 21, 22...... illuminator, light source (LED illumination)
26 visual field measuring means (stimulus presenter)
27 visual field measuring means (visual field judger)
29 brightness computer, installation mode computer, illumination controller (illumination controller)
31 lens-brightness database
CT measurement position (center)
P1 set position
P2 stored position

## Claims

1. A perimeter having:
a visual field measurer that measures a visual field of a subject eye when the subject eye perceives stimuli projected in order at different positions on a projection member:
a fixation state monitor that monitors a fixation state of the subject eye at a time of perimetry;
the fixation state monitor having an illuminator that lights the subject eye with a brightness necessary for monitoring the fixation state of the subject eye; and
a visibility corrector that corrects visibility of the subject eye; the perimeter further comprising:
a lens holder portion provided on the visibility corrector so as to detachably attach and exchange and combine one or more correction lenses;
a memory that stores a lens-brightness database showing brightness of the illuminator when lighting the subject eye with almost the same brightness according to an installation mode of the correction lens installed in the lens holder portion;
an installation mode computer that computes the installation mode of the correction lens with respect to the visibility corrector based upon correction information of the subject eye;
a brightness computer that computes and determines the brightness of the illuminator in association with the installation mode of the correction lens computed through the installation mode computer based upon the lens-brightness database; and
an illumination controller that lights the subject eye through the illuminator based upon the brightness of the illuminator that was computed and determined through the brightness computer.

2. The perimeter according to claim 1, wherein the visibility corrector is provided being free to be moved with respect to the projection member between a set position corresponding to a perimetry position of the subject eye and a stored position retracted to an outer periphery of the projection member; further comprising a position detector that detects whether the visibility corrector is at the set position or the stored position.

3. The perimeter according to claim 2, wherein the illuminator is comprised of two or more light sources that are located respectively different positions, and the illumination controller controls to light the subject eye when the visibility corrector is positioned at the set position by more light sources rather than a case where the visibility corrector is positioned at the stored position.

4. The perimeter according to claim 2, wherein the illuminator has at least two light sources,
at least one of the light sources is positioned at a position where the subject eye can be lighted via a lens for correcting visibility that is held by the visibility corrector when the visibility corrector is used at the set position;
at least another one light source is positioned at a position where the subject eye can be directly lighted without passing through the lens for correcting visibility that is held by the visibility corrector even if the visibility corrector is used at the set position.

5. The perimeter according to claim 1, 3 or 4, wherein the illuminator has at least three light sources, and at least one of the light sources is located on a lower hand of a center of the projection member and at least two light sources are positioned at both sides of a lower hand of the projection member.

6. The perimeter according to claim 2, wherein the illumination controller lights the subject eye in a state that the brightness of the light source when the visibility corrector is positioned at the set position is made higher than one when the visibility corrector is positioned at the stored position.

7. The perimeter according to claim 1, wherein the lens-brightness database shows the brightness of the illuminator in association with presence of the lens installed in the lens holder portion, a kind and a refractive index of the lens.

8. The perimeter according to claim 1, wherein the lens holder portion is provided with a sensor that detects the kind, a degree and the number of the correction lenses installed in the lens holder portion and outputs the detected to the outside, and the installation mode computer computes an installation mode of the correction lens based upon a signal from the sensor.

9. The perimeter according to claim 1, wherein the illuminator has at least two light sources, and the illumination controller selectively drives the light sources when lighting the subject eye.

10. The perimeter according to claim 1, wherein the illuminator has at least two light sources, and the illumination controller drives the light sources with different brightness when lighting the subject eye.
